# EUROPEAN PATENT APPLICATION

(11) **EP 4 748 836 A1**
(43) Date of publication of application: **27.05.2026**
(21) Application number: 24214654.6
(22) Date of filing: 21.11.2024
(51) Int. Cl.: C07D 313/08, C08G 61/00, C08G 63/00

(54) **LIGNIN DERIVED MONOMERS**

(71) Applicant: BASF SE, 67056 Ludwigshafen am Rhein (DE); Universiteit Utrecht Holding B.V., 3584 CS Utrecht (NL)
(72) Inventor: RIDDELL, Luke Alexander, 3584 CG Utrecht (NL); KOFFIJBERG, Eline Marijtie, 3584 CG Utrecht (NL); MEIRER, Florian, 3584 CG Utrecht (NL); LINDNER, Jean-Pierre Berkan, 67056 Ludwigshafen am Rhein (DE); BRUIJNINCX, Pieter Cornelis Antonius, 3531 TJ Utrecht (NL)
(74) Representative: Meissner Bolte Partnerschaft mbB

(57) **Abstract**

A compound of formula (I) 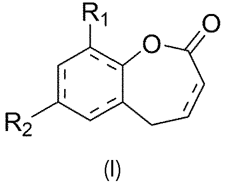 wherein
R₁ and R₂
are independently H, C₁-C₁₈-alkyl or C₁-C₁₈-alkoxy, which alkyl and/or alkoxy is unsubstituted or substituted with at least one substituent R₃,
R₃
is independently halogen, hydroxyl, C₁-C₁₈-alkoxy, Cs-Cs-cycloalkyl, Cs-Cs-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted,
- - -
denotes a single bond or a double bond, and

with the proviso that the compound in which R₁ is H and R₂ is H is excluded
is a lignin derived monomer that is capable of self-polymerization. Polymers are obtained from the compound of formula (I) where the 7-membered ring contains an olefinic unsaturation by ring-opening metathesis polymerization. Polymers are obtained from the compound of formula (I) where the 7-membered ring contains no olefinic unsaturation by ring-opening polymerization.

## Description

The present invention relates to lignin derived monomers that are capable of self-polymerization, a method for preparing the same, a polymer comprising repeating units derived from the lignin derived monomers, and a method for preparing said polymer.

Phenol-derived compounds (phenolics) are utilised ubiquitously for the production of, e.g., polyesters. Typically, the vast majority of the phenol used for this purpose is fossil-derived. In order to reduce the demand for phenol and, simultaneously, reduce the demand of fossil resources, alternatives for phenolics having comparable reactivity are of special interest.

Preferably, the phenol-derived compounds should be based on renewable resources. Lignin is among the most abundant biopolymers in nature. Lignin is also a major waste product in cellulose-driven industries, e.g. paper & pulping, bulk chemicals, bioethanol etc. Thus, lignin serves as a potential source of renewable, functionalised aromatics which may, e.g., replace phenol. Structurally, lignin is a highly heterogeneous and complex biopolymer matrix. Depolymerisation of typical industrial (technical) lignins has been described in the literature and gives access to monophenolics. Besides depolymerization, "lignin-first" technologies such as reductive catalytic fractionation (RCF) have provided an alternative way to access monophenolics. Propylated guaiacol is a typical monomethoxylated phenol obtained as the primary products in relatively high quantities through such technologies.

Some phenol-derived bicyclic compounds depicted below have been reported in the literature, for example in H.-Z. Fan et al., Polym. Chem. 2023, 14, 747; H. J. Kim et al., ACS Macro Lett. 2020, 9, 96; V. Makwana et al., Polym. Degrad. Stab. 2019, 169, 108984; and Y.-M. Tu et al., J. Am. Chem. Soc. 2021, 143, 20591:

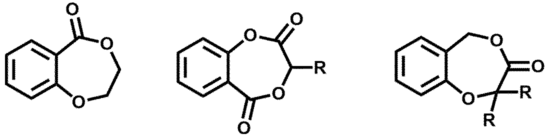

However, these compounds lack a lignin derived substitution pattern of the phenolic ring, e.g., alkyl and/or methoxy residues, and therefore, such compounds are usually almost entirely obtained from from fossil-derived carbon. Translating these approaches towards utilisation of biorenewable feedstocks or even waste would be highly desirable for improving the sustainability of these compounds. Such examples of these types of monomers, however, are thus far largely lacking.

It is therefore an object of the present invention to provide suitable renewable phenol-derived compounds, especially for phenol-derived compounds which can be used for the production of polyesters such as unsaturated polyesters.

Furthermore, the current materials sector is heavily reliant on polyolefins due to their superior material properties, finding applications in, e.g. food packaging, construction, consumer goods, high-end scientific/medical equipment etc. Their remarkable resistance to mechanical and chemical wear is another key factor which allows them to be so well suited for such a diverse range of applications. However, this also imparts them with an intrinsic lack of chemical recyclability or (bio)degradation and has led to systemic plastic pollution of the environment. The resilience of these materials to wear is primarily a result of their highly unreactive C-C backbone. The majority of plastic recycling is through mechanical means, however these are often high-cost and the recycled materials typically exhibit a loss in performance, and thus these methods are often considered to be a form of downcycling. There is therefore burgeoning industrial and fundamental interest in 'closed-loop' recycling of polymers at end-of-life. For this, the polymer must be suitable to undergo the chemical transformations back to the monomer, requiring reactive handles within the polymer backbone that can be selectively and reversibly cleaved. Polycondensates are intrinsically most suited for this, with polyesters being the most popular class of these materials. However, polyesters do not efficiently depolymerise under environmental conditions and are thus not biodegradable leading to long-time environmental pollution.

It is therefore a further object of the present invention to provide suitable polymers comprising repeating units derived from the above-mentioned monomers, which polymers allow for a good recyclability and/or (bio)degradation.

The object(s) of the invention is solved by a compound of formula (I) 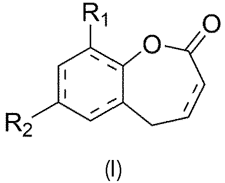 wherein
- R₁ and R₂: are independently H, C₁-C₁₈-alkyl or C₁-C₁₈-alkoxy, which alkyl and/or alkoxy is unsubstituted or substituted with at least one substituent R₃,
- R₃: is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted,
- - - -: denotes a single bond or a double bond, and
with the proviso that the compound in which R₁ is H and R₂ is H is excluded.

The inventive compounds having a fused 6- and 7-membered ring provide a variety of advantages. The inventive compounds can advantageously be obtained from, e.g., 4-propyl guaiacol which is a renewable compound, e.g. being obtainable from lignin depolymerization. The inventive compounds can be polymerized under ring opening methathesis polymerization (ROMP) conditions to obtain unsaturated polyesters. The resulting polymers contain relatively labile ester bonds resulting in an advantageous recyclability and/or (bio)degradation enabling the use in applications which require fast and efficient decomposition under controlled conditions like packaging solutions, foils for agricultural applications or functional polymers in home and personal care.

In an embodiment, R₁ and R₂ are independently H, C₁-C₁₂-alkyl or C₁-C₁₂-alkoxy, preferably H, C₁-C₃-alkyl or C₁-C₃-alkoxy.

In an embodiment, R₃ is alkoxy which alkoxy may be substituted with at least one substituent selected from C₁-C₁₈-alkoxy and halogen, preferably C₁-C₁₂-alkoxy and halogen, more preferably C₁-C₃-alkoxy and halogen.

In an embodiment, R₃ is cycloalkyl, cycloalkoxy, heterocycloalkyl, heterocycloalkoxy, aryl, aryloxy, hetaryl or hetaryloxy which cycloalkyl, cycloalkoxy, heterocycloalkyl, heterocycloalkoxy, aryl, aryloxy, hetaryl or hetaryloxy may be substituted with at least one substituent selected from C₁-C₁₈-alkyl, C₁-C₁₈-alkoxy and halogen, preferably C₁-C₁₂-alkyl, C₁-C₁₂-alkoxy and halogen, more preferably C₁-C₃-alkyl, C₁-C₃-alkoxy and halogen.

In an especially preferred embodiment,
R₁ is H or methoxy, and
R₂ is H, methyl, ethyl or n-propyl, preferably H or n-propyl.

In the compound of formula (I), - - - denotes a single bond or a double bond. Therefore, the compound of formula (I) may denote a compound of formula (Ia), (Ib), (Ic) and/or (Id):

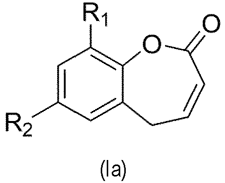

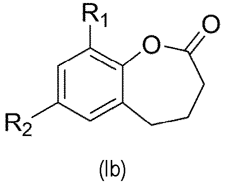

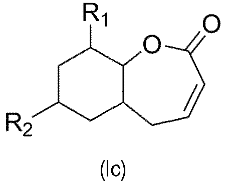

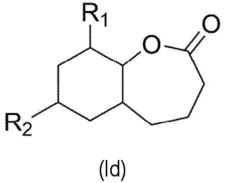

Among (la), (lb), (Ic) and (Id), preference is given to (Ia), (lb) and (Id).

The invention further relates to a method for preparing a compound of formula (Ia) 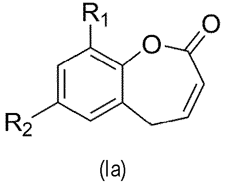 wherein
- R₁ and R₂: are independently H, C₁-C₁₈-alkyl or C₁-C₁₈-alkoxy, which alkyl and/or alkoxy is unsubstituted or substituted with at least one substituent R₃, and
- R₃: is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted,
with the proviso that the compound in which R₁ is H and R₂ is H is excluded, the method comprising subjecting a compound of formula (Ila) 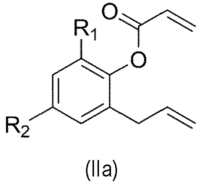 to reaction conditions conducive of a ring-closing metathesis reaction to obtain the compound of formula (la).

Reaction conditions conducive of a ring-closing metathesis (RCM) reaction are known by the skilled person.

Typically, reaction conditions conducive of a ring-closing metathesis reaction comprise adding a catalyst ("metathesis catalyst") which is active in ring-closing metathesis reactions. Exemplary metathesis catalysts are selected from (C-1), (C-2) and (C-3):

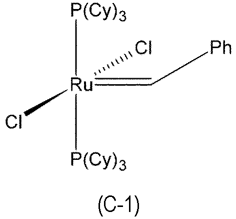

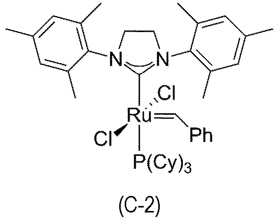

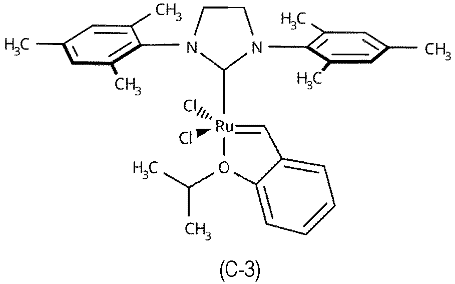

Generally, (C-1) is referred to as "Grubbs' first generation catalyst" or "Grubbs I"; (C-2) is referred to as "Grubbs' second generation catalyst" or "Grubbs II"; and (C-3) is referred to as "Grubbs' third generation catalyst" or "Grubbs-Hoveyda II".

Among (C-1), (C-2) and (C-3), catalyst (C-2) is preferred as ring-closing metathesis catalyst.

The method may involve adding the metathesis catalyst in an amount in the range of from 0.01 to 5 mol-%, preferably 0.1 to 3 mol-%, more preferably 0.5 to 1 mol-%, relative to the molar amount of the compound of formula (Ila).

Suitably, the method may involve carrying out the ring-closing metathesis reaction in a solvent. The solvent may be selected from dichloromethane, chloroform, ethyl acetate, tetrahydrofuran and toluene. Suitable concentrations of the compound of formula (Ila) in the solvent are in the range of from 1 to 100 mmol/L, preferably 1.5 to 50 mmol/L, more preferably 2 to 10 mmol/L.

Suitably, the method may involve carrying out the ring-closing metathesis reaction under an inert gas atmosphere. Suitable inert gases are selected from argon and nitrogen.

Suitably, the method may involve carrying out the ring-closing metathesis reaction at a temperature in the range of from 30 to 100 °C, preferably 40 to 90 °C, more preferably 50 to 80 °C.

Suitably, the method may involve carrying out the ring-closing metathesis reaction at a reaction time of 1 to 24 h, preferably 1 to 5 h.

In an embodiment, the method further comprises reacting a compound of formula (Illa) 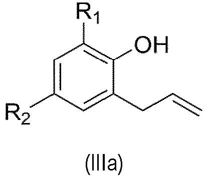 with a compound of formula (IVa) 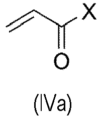 wherein
X is a leaving group, preferably halogen such as Cl or Br, preferably Cl, or acryloyloxy,
to obtain the compound of formula (Ila).

In an especially preferred embodiment, the compound of formula (lVa) is acryloyl chloride.

Suitable reaction conditions for carrying out the esterification reaction to obtain the compound of formula (Ila) from the compound of formula (Illa) are known by the skilled person.

Suitably, the method comprises reacting the compound of formula (Illa) with excess amounts of the compound of formula (lVa). Preferably, the method comprises reacting about 1 equivalent of the compound of formula (Illa) with about 2 equivalents of the compound of formula (IVa).

Typically, the method may involve adding a base. The base is added to quench by-products typically formed in the esterification reaction. For example, in the case X in the compound of formula (IVa) is Cl, acidic hydrogen chloride may be formed as by-product. The base may be selected from trimethylamine, triethylamine, 4-dimethylaminopyridine (DMAP) and 1-methylimidazol. Triethylamine is especially preferred. The method may involve adding the base in an amount in the range of from 100 to 500 mol-%, preferably 200 to 400 mol-%, relative to the molar amount of the compound of formula (Illa).

Suitably, the method may involve carrying out the esterification reaction in a solvent. The solvent may be selected from dichloromethane, chloroform, ethyl acetate, tetrahydrofuran and toluene. Suitable concentrations of the compound of formula (Illa) in the solvent are in the range of from 0.1 to 2 mol/L, preferably 0.2 to 1.5 mol/L, more preferably 0.21 to 1.3 mol/L.

Suitably, the method may involve carrying out the esterification reaction under an inert gas atmosphere. Suitable inert gases are selected from argon and nitrogen.

Suitably, the method may involve carrying out the esterification reaction at a temperature in the range of from 0 °C to room temperature.

Suitably, the method may involve carrying out the esterification reaction at a reaction time of 1 to 48 h, preferably 1 to 24 h.

In an embodiment, the method further comprises subjecting a compound of formula (Va) 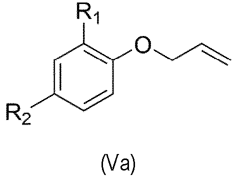 to reaction conditions conducive of a Claisen rearrangement to obtain the compound of formula (IIIa).

Reaction conditions conducive of a Claisen rearrangement, i.e. a thermally induced [3,3]-sigmatropic rearrangement, are known by the skilled person.

Suitably, the method may involve carrying out the Claisen rearrangement without a solvent ("neat").

Suitably, the method may involve carrying out the Claisen rearrangement under an inert gas atmosphere. Suitable inert gases are selected from argon and nitrogen.

Suitably, the method may involve carrying out the Claisen rearrangement at a temperature in the range of from 150 to 200 °C, preferably 160 to 190 °C.

Suitably, the method may involve carrying out the Claisen rearrangement at a reaction time of 12 to 72 h.

In an embodiment, the method further comprises reacting a compound of formula (Vla) 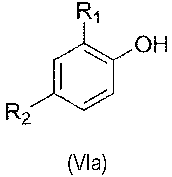 with a compound of formula (VIIa) 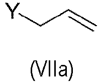 wherein
Y a is leaving group, preferably halogen such as Cl or Br, preferably Br, mesylate or tosylate,
to obtain the compound of formula (Va).

Suitable reaction conditions for carrying out the reaction, i.e. the Williamson-type etherification reaction, to obtain the compound of formula (Va) from the compound of formula (Vla) are known by the skilled person.

In an especially preferred embodiment, the compound of formula (Vlla) is allyl bromide.

Suitably, the method comprises reacting the compound of formula (Vla) with excess amounts of the compound of formula (Vila). Preferably, the method comprises reacting about 1 equivalent of the compound of formula (Vla) with about 1.2 equivalents of the compound of formula (Vlla).

Typically, the Williamson-type etherification reaction may involve adding a base. The base is added to quench by-products typically formed in the Williamson-type etherification reaction. For example, in the case Y in the compound of formula (Vlla) is Br, acidic hydrogen bromide may be formed as by-product. The base may be selected from alkali metal bases such as sodium carbonate, potassium carbonate, sodium bicarbonate, potassium bicarbonate, sodium hydroxide and potassium hydroxide. Potassium carbonate is especially preferred. The base may be added in an excess amounts. For example, the method comprises reacting 1.1 to 3 equivalents, preferably about 2 equivalents, of the base, per equivalent of the compound of formula (VIIa).

Suitably, the method may involve carrying out the Williamson-type etherification reaction in a solvent. The solvent may be selected from dichloromethane, chloroform, ethyl acetate, tetrahydrofuran and toluene, and acetone. Suitable concentrations of the compound of formula (IIIa) in the solvent are in the range of from 0.15 to 0.25 mol/L.

Suitably, the method may involve carrying out the Williamson-type etherification reaction at a temperature in the range of from 50 to 100 °C.

Suitably, the method may involve carrying out the Williamson-type etherification reaction at a reaction time of 12 to 168 h.

The four-step reaction sequence starting from the compound of (Vla) via (Va), (Illa), (Ila) to obtain the compound of formula (Ia) can be summarized as follows:

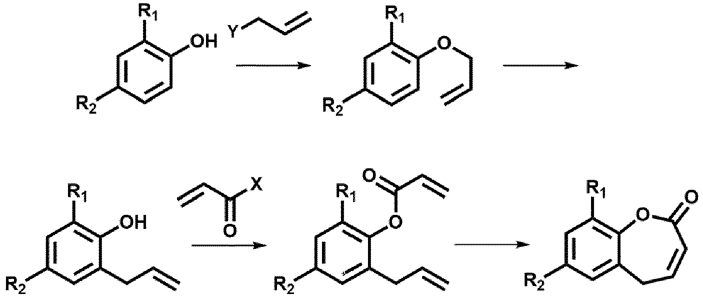

In a preferred embodiment, the method comprises depolymerizing lignin to obtain the compound of formula (Vla). For example, the compound of formula (Vla) may be 4-propyl guaiacol. Lignin is one renewable resource that shows promise as a desirable alternative to petroleum feedstocks, largely due to its abundance as a byproduct of pulp and paper refining. This advantageous method allows for utilizing a compound of formula (Vla) obtained from the renewable resource lignin.

Hydrogenating the compound of formula (la) may give rise to the above shown compound of formula (lb), (Ic) and/or (Id). Thus, in a preferred embodiment, the method comprises hydrogenating the compound of formula (Ia) to obtain the compound of formula (lb)

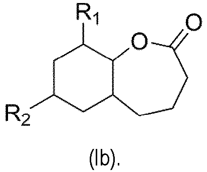

Suitable reaction conditions for hydrogenating the compound of formula (la) are known by the skilled person.

Typically, the hydrogenation reaction is catalyzed by a hydrogenation catalyst. Therefore, the method may involve adding a hydrogenation catalyst. The hydrogenation catalyst may be selected from Pd/C, Pt/C, Ni/Al₂O₃, and Cu/Al₂O₃. Pd/C is especially preferred. The hydrogenation catalyst may be added in an amount in the range of from 0.1 to 1 eq., relative to the molar amount of the compound of formula (Ia).

Generally, a hydrogenation reaction requires the presence of a source of hydrogen. Suitably, the source of hydrogen may be hydrogen gas. The method may involve carrying out the hydrogenation reaction in the presence of hydrogen gas, preferably at a hydrogen pressure in the range of from 1 to 100 bar.

Suitably, the method may involve carrying out the hydrogenation reaction in a solvent. The solvent may be selected from dichloromethane, chloroform, ethyl acetate, tetrahydrofuran and toluene. Suitable concentrations of the compound of formula (Ia) in the solvent are in the range of from 0.1 to 2 mol/L.

Suitably, the method may involve carrying out the hydrogenation reaction at a temperature in the range of from 20 to 100 °C.

Suitably, the method may involve carrying out the hydrogenation reaction at a reaction time of 12 to 24 h.

The invention further relates to a polymer comprising repeating units of formula (Villa) 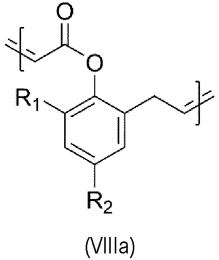 wherein
- R₁ and R₂: are independently H, C₁-C₁₈-alkyl or C₁-C₁₈-alkoxy, which alkyl and/or alkoxy is unsubstituted or substituted with at least one substituent R₃,
- R₃: is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted, and
- - - -: denotes a single bond or a double bond.

The polymer comprising repeating units of formula (Villa) in which - - - is a double bond may be obtained by polymerizing the compound of formula (la) as described in further detail in the following.

Therefore, the invention further relates to a method for preparing the polymer of formula (Villa) in which - - - is a double bond as shown above. The method comprises subjecting the compound of formula (la) as shown above reaction conditions conducive of a ring-opening metathesis polymerization (ROMP) reaction to obtain the polymer comprising repeating units of formula (Villa) in which - - - is a double bond.

Reaction conditions conducive of a ring-opening metathesis polymerization (ROMP) reaction are known by the skilled person.

Typically, reaction conditions conducive of a ring-opening metathesis polymerization reaction comprise adding a catalyst ("metathesis catalyst") which is active in ring-opening metathesis polymerization reactions. Exemplary metathesis catalysts are selected from (C-1), (C-2) and (C-3):

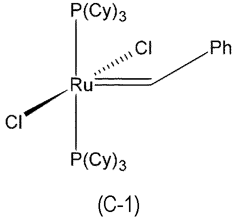

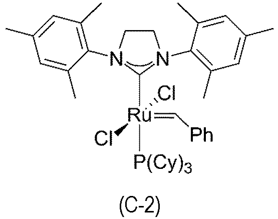

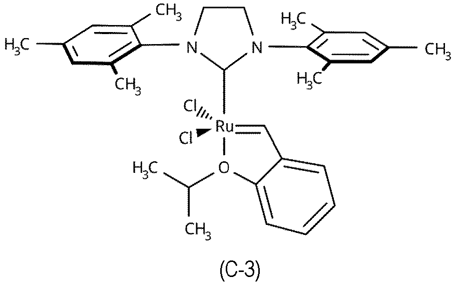

Among (C-1), (C-2) and (C-3), catalyst (C-3) is preferred as ring-opening metathesis polymerization catalyst.

The method may involve adding the metathesis catalyst in an amount in the range of from 0.1 to 10 mol-%, relative to the molar amount of the compound of formula (la).

Suitably, the method may involve carrying out the ring-opening metathesis polymerization reaction in a solvent. The solvent may be selected from dichloromethane, chloroform, ethyl acetate, tetrahydrofuran and toluene. Suitable concentrations of the compound of formula (Ila) in the solvent are in the range of from 1 to 10 mol/L.

Suitably, the method may involve carrying out the ring-opening metathesis polymerization reaction under an inert gas atmosphere. Suitable inert gases are selected from argon and nitrogen.

Suitably, the method may involve carrying out the ring-opening metathesis polymerization reaction at a temperature in the range of from 40 to 80 °C.

Suitably, the method may involve carrying out the ring-opening metathesis polymerization reaction at a reaction time of 1 to 24 h.

In an embodiment, the method comprises hydrogenating the compound of formula (Villa) in which - - - is a double bond to obtain a compound of formula (Villa) in which - - - is a single bond.

Suitable reaction conditions for hydrogenating the compound of formula (Villa) in which - - - is a double bond are known by the skilled person.

Typically, the hydrogenation reaction is catalyzed by a hydrogenation catalyst. Therefore, the method may involve adding a hydrogenation catalyst. The hydrogenation catalyst may be selected from Pd/C, Pt/C, Ni/Al₂O₃, and Cu/Al₂O₃. Pd/C is especially preferred.

Generally, a hydrogenation reaction requires the presence of a source of hydrogen. Suitably, the source of hydrogen may be hydrogen gas. The method may involve carrying out the hydrogenation reaction in the presence of hydrogen gas, preferably at a hydrogen pressure in the range of from 1 to 100 bar.

Suitably, the method may involve carrying out the hydrogenation reaction in a solvent. The solvent may be selected from dichloromethane, chloroform, ethyl acetate, tetrahydrofuran and toluene.

Suitably, the method may involve carrying out the hydrogenation reaction at a temperature in the range of from 20 to 100 °C.

Suitably, the method may involve carrying out the hydrogenation reaction at a reaction time of 12 to 24 h.

The invention further relates to a polymer comprising repeating units of formula (IX) 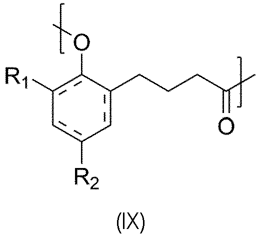 wherein
- R₁ and R₂: are independently H, C₁-C₁₈-alkyl or C₁-C₁₈-alkoxy, which alkyl and/or alkoxy is unsubstituted or substituted with at least one substituent R₃,
- R₃: is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted, and
- - - -: denotes a single bond or a double bond.

The polymer comprising repeating units of formula (IX) may be obtained by polymerizing the compound of formula (lb) and/or (Id), preferably the compound of formula (Id), as described in further detail in the following.

Therefore, the invention further relates to a method for preparing the polymer comprising repeating units of formula (IX) as shown above. The method comprises subjecting a compound of formula (I') 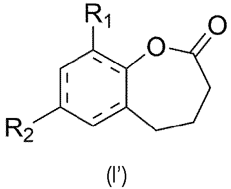 wherein
- R₁ and R₂: are independently H, C₁-C₁₈-alkyl or C₁-C₁₈-alkoxy, which alkyl and/or alkoxy is unsubstituted or substituted with at least one substituent R₃,
- R₃: is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted, and
- - - -: denotes a single bond or a double bond, preferably a single bond,
to reaction conditions conducive of a ring-opening polymerization reaction to obtain the polymer comprising repeating units of formula (IX).

Reaction conditions conducive of a ring-opening polymerization reaction are known by the skilled person.

Typically, reaction conditions conducive of a ring-opening polymerization reaction comprise adding a ring-opening polymerization catalyst. The ring-opening polymerization catalyst may be selected from ring-opening polymerization metal catalysts such as dibutyltin dilaurate, titanium tetraisopropoxide or methyl aluminium salen complexes (MeAl[salen]); or from ring-opening polymerization base catalysts such as 1,4-diazabicyclo(2.2.2)octan (DABCO), 1,5,7-triazabicyclo(4.4.0)dec-5-en (TBD) or 1,8-diazabicyclo(5.4.0)undec-7-ene (DBU).

The method may involve adding the ring-opening polymerization catalyst in an amount in the range of from 0.1 to 1 eq., relative to the molar amount of the compound of formula (I').

Suitably, the method may involve carrying out the ring-opening polymerization reaction in a solvent. The solvent may be selected from dichloromethane, chloroform, ethyl acetate, tetrahydrofuran and toluene. Suitable concentrations of the compound of formula (IIa) in the solvent are in the range of from 1 to 10 mol/L.

Suitably, the method may involve carrying out the ring-opening polymerization reaction under an inert gas atmosphere. Suitable inert gases are selected from argon and nitrogen.

Suitably, the method may involve carrying out the ring-opening polymerization reaction at a temperature in the range of from 40 to 80 °C.

Suitably, the method may involve carrying out the ring-opening polymerization reaction at a reaction time of 1 to 24 h.

The present invention is further illustrated by the examples that follow.

### Examples

### Materials

Solvents and commercially available reagents were used as received unless stated otherwise. 2-methoxy-4-propylphenol (>99%), 4-propylphenol (99%), acryloyl chloride (≥ 97%), 1,3-diaminopropane (>90%), 3,5-di-tert-butyl-2-hydroxybenaldehyde (>99%) and Grubbs catalyst, 2nd generation were obtained from Sigma Aldrich. Allyl bromide (99%) (stabilised), guaiacol (>99%), 2-allylphenol (>98%), K₂CO₃ (98%), NEt₃ (99%), benzyl alcohol (99%), MeAl₃ (1.0 M solution in heptane), 3-bromopyridine (99%), ethyl vinyl ether (99%) (stabilised) and MgSO₄ (99%) were obtained from Thermo Fisher Scientific. Sn(Oct)₂ (>85%) from TCI. Acetone (100.0%), DCM (100%), ethyl acetate (≥ 99%) and methanol (100.0%) were obtained from VWR chemicals. Technical grade DCM (99.9%), peptide synthesis grade DCM (99.9%), petroleum ether 40-60 (100%), AR toluene (99.7%), and peptide synthesis grade N,N-Dimethylformamide (>99.8%) were obtained from Biosolve. Silica (gel) (0.060-0.200 nm, 60 A) and basic aluminium oxide (50-200 µm, 60 A) and molecular sieves (3 Å and 4 Å) were obtained from Thermo Fisher Scientific. CDCl₃ (≥99.8%) was obtained from Sigma Aldrich. CD₃CN (99.8%) was obtained from Cambridge Isotope Laboratories.

Peptide synthesis grade DCM and AR toluene were dried using an MBRAUN MB SPS-79 system, degassed, and stored over activated molecular sieves prior to use. The molecular sieves (3 Å for DCM and 4 Å for toluene) were activated by heating to 250 °C for at least 12 h prior to use. It must be noted that extensive purification was performed for the DCM, first through a solvent purification system (SPS), followed by storage over 3 Å molecular sieves for at least 16 h. These steps were found to be essential for full conversion during the RCM. As a consequence of this more laborious solvent preparation, this led us to endeavour to recover the majority of the DCM post-RCM, via careful distillation from our product. In doing so, of the 1 L of DCM used in this reaction step, only (approximately) 50-100 mL were not recovered.

Water content in the solvents were titrated with a Karl Fischer Coulometer. Reactions which were performed under inert conditions were carried out in a nitrogen atmosphere using standard Schlenk techniques or a nitrogen filled MBRAUN glovebox. All glassware used in such reactions was dried for a minimum of four hours and up to overnight, prior to use. Benzyl alcohol was purified by distillation over CaH₂. Grubbs 3^{rd} generation catalyst was prepared from Grubbs 2^{nd} generation catalyst and 3-bromopyridine according to a literature protocol.

### Equipment

All NMR measurements were performed at 25 °C on either a Varian VNMRS-400 NMR spectrometer with a AutoX probe and Agilent ProTune system, or a 400 MHz Jeol EZCL G system equipped with an HFX probe. Acquired data was analysed and processed using Mestrenova (version 14.2.1-27684).

Fourier-Transform Infrared (FTIR) analysis was performed on a PerkinElmer Spectrum Two spectrometer, equipped with a Universal ATR accessory with a diamond crystal and LiTaO₃ detector. Spectra were acquired across the range of 4000-600 cm⁻¹ with a resolution of 4 cm⁻¹ using 8 scans.

ESI-MS were recorded on an Advion Expression L CMS with MeCN as carrier solvent.

Polymers were separated from the solvent by using the SIGMA 2-16 P Centrifuge.

Melting points were determined with Stuart SMP3 Melting Point Apparatus, with a heating rate of 5 °C per minute.

MDSC measurements were performed using a TA instruments Trios DSC with samples contained in Tzero low-mass aluminium pans, and the data was analysed within the TA instruments Trios software (v5.7.1.74).

GPC measurements were performed using a modular Shimadzu system comprising an SCI-40 controller module, DGU-203 degassing unit, SIL-40 autosampler, CTO-40C column oven, and RID-20A refractive index detector. The system featured an Agilent Technologies PLgel 5µm Guard 50 x 7.5 mm guard column followed by two Agilent Technologies PLgel 5µm MIXED-D 300 x 7.7 mm columns in series. Data analysis was performed using Shimadzu's Labsolutions GPC software.

TGA measurements were carried out on a TA Instruments TGA Q50 Thermographic Analyzer in a nitrogen atmosphere on a platinum pan. The resulting data was analysed using TA instruments Universal Analysis 2000.

### Methods

### Nuclear Magnetic Resonance (NMR)

The chemical shifts (δ) are given in ppm relative to tetramethylsilane (TMS) and using residual solvent peaks as a reference. The ¹H NMR data contains the shift in ppm, multiplicity, coupling constant (J) in Hz and quantity of protons. The multiplicity is indicated with abbreviations of: singlet (s), doublet (d), triplet (t), quartet (q), pentet (p), and multiplet (m). In all cases, an arbitrary quantity of sample (approximately 5-20 mg) was dissolved in the NMR solvent within a 5 mm OD NMR tube immediately prior to analysis. All measurements were performed at 298 K. For ¹H NMR measurements, a standard ¹H pulse sequence was used with 8 scans. For ¹³C-APT NMR measurements, inverse-gated decoupling of ¹H was applied, and the number of scans varied between 128-2560+. All ¹D NMR spectra were processed in the same way unless otherwise specified: A 3^{rd} order polynomial baseline correction was applied, followed by automatic phase correction.

### Gel Permeation Chromatography (GPC)

Samples were dissolved in N,N-dimethylformamide (DMF) (1 mg mL⁻¹) and were filtered with a 0.45 µm PTFE syringe filter before GPC analysis. The sample injection volume was 10 µL. In certain samples, extra DMF was introduced as the sample volumes were too small after filtration, leading to an increased injected volume of 30 µL per measurement. The measurements were run at a flow rate of 1 mL min⁻¹ at 65 °C with pressures between 85 to 92 bar. The molecular weight determinations were calibrated with polystyrene standards (Mn = 4k, 7k, 13k, 20k, 50k, 100k, 200k, 300k g mol⁻¹). The calibration set was measured prior to each sample measurement. It is important to note, that since polystyrene differs in structure from the measured polymer, its application as proxy calibration standard suggests that caution should be taken when interpreting the reported molecular weights concerning their absolute molecular weight. Measurements were taken at least in duplo, and in some cases in triplo for each sample. The mean values for Mₙ and M_{w} were then taken.

### Modulated Differential Scanning Calorimetry (MDSC)

1 to 5 mg of sample was accurately weighed into a Tzero Low-Mass aluminium pan, fitted with a Tzero lid. After loading the pan into the DSC cell, the sample was equilibrated to 25 °C. Subsequently, two heating cycles were performed, with all steps performed under N₂ flow: In the first cycle, the sample was heated to 105 °C at a rate of 10 °C min⁻¹, followed by a 10-minute isothermal hold, cooling to -40 °C at 10 °C min⁻¹, and finally equilibration of the sample at -40 °C to anneal the sample, remove any residual solvents and erase thermal history of the sample. Data acquisition occurred during the second heating cycle, involving a temperature ramp from -40 °C to 200 °C at 3 °C min⁻¹ with a sinusoidal temperature modulation of 0.66 °C per 50 s. Duplicate measurements were taken for each sample and the average value was taken.

### Thermogravimetric Analysis (TGA)

Approximately 5 mg of sample was weighed onto a platinum pan. After loading the pan into the TGA, the sample was heated from 20 to 1000 °C at a rate of 20 °C min⁻¹ under N₂ flow.

### Elemental Analysis (EA)

Elemental Analysis was performed by MEDAC LTD Analytical and Chemical Consultancy Services in Surrey, United Kingdom. Duplicate measurements were taken for each sample. Both of these values, the averages, and the calculated standard deviations have been reported below. It is generally accepted that measured values for carbon, hydrogen and nitrogen should fall within the range ± 0.4% compared to the calculated values, as outlined by the submission guidelines of many journals.

### Syntheses

### General Procedure 1 to obtain the compound of formula (Va): Williamson-type Ether Synthesis

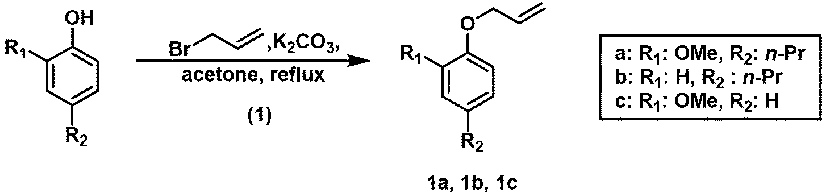

The phenolic compound (1 eq., 0.20 M) and K₂CO₃ (2 eq., 0.39 M) were added to a round bottom flask with acetone. While stirring, allyl bromide (1.2 eq., 0.23 M) was added with a syringe and the reaction mixture was heated to reflux for 24 h up to 7 days, after which the mixture was cooled to room temperature. The mixture was filtered and the residue washed with acetone. The filtrate was purified by a basic alumina plug, dried by MgSO₄, filtered, and reduced in vacuo, resulting in the desired product.

### 1-(Allyloxy)-2-methoxy-4-propylbenzene (1a)

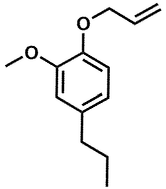

2-methoxy-4-propylphenol (13.3 g, 79.9 mmol), K₂CO₃ (20.0 g, 144.7 mmol), allyl bromide (8 mL, 92.4 mmol) in acetone (400 mL) were reacted for 5 days, resulting in a light-yellow oil (16.4 g, 98% yield) which was isolated without further purification.

¹H NMR (400 MHz, CDCl₃): δ 6.80 (d, J = 8.0 Hz, 1H), 6.70 (m, 2H), 6.09 (ddt, J = 17.3, 10.7, 5.4 Hz, 1H), 5.39 (dq, J = 17.3, 1.6 Hz, 1H), 5.26 (dq, J = 10.5, 1.4 Hz, 1H), 4.59 (dt, J = 5.5, 1.5 Hz, 2H), 3.87 (s, 3H), 2.53 (t, J = 7.9 Hz, 2H), 1.63 (h, J = 7.6 Hz, 2H), 0.94 (t, J = 7.3 Hz, 3H) ppm.

¹³C-APT NMR (101 MHz, CDCl₃): δ 149.38, 146.13, 136.01, 133.83, 120.30, 117.87, 113.67, 112.36, 70.20, 56.01, 37.83, 24.86, 13.98 ppm.

IR-ATR (cm⁻¹): 2957.63, 2929.73, 2870.08, 1703.90, 1648.97, 1606.39, 1589.33, 1511.40, 1464.70, 1417.75, 1259.71, 1226.12, 1157.59, 1140.12, 1024.60, 995.36, 924.45, 801.04.

### 1-(Allyloxy)-4-propylbenzene (1b)

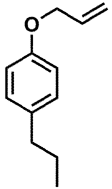

4-propylphenol (2.0 g, 15.0 mmol), K₂CO₃ (4.1 g, 29.8 mmol), allyl bromide (1.5 mL, 17.3 mmol) in acetone (100 mL) were reacted for a time period of 7 days, resulting in a light-yellow oil (2.7 g, 103% yield; residual solvent and grease) which was isolated without further purification.

¹H NMR (400 MHz, CDCl₃): δ 7.12 - 7.04 (m, 2H), 6.88 - 6.80 (m, 2H), 6.06 (ddt, J = 17.3, 10.6, 5.3 Hz, 1H), 5.41 (dq, J = 17.3, 1.6 Hz, 1H), 5.27 (dq, J = 10.5, 1.5 Hz, 1H), 4.52 (dt, J = 5.3, 1.6 Hz, 2H), 2.53 (t, J = 7.8 Hz, 2H), 1.61 (h, J = 7.3 Hz, 2H), 0.93 (t, J = 7.3 Hz, 3H) ppm.

¹³C-APT NMR (101 MHz, CDCl₃): δ 156.82, 135.17, 133.73, 129.43, 117.63, 114.65, 69.03, 37.30, 29.42, 24.89, 13.92 ppm.

IR-ATR (cm⁻¹): 3028.54, 2959.71, 2928.93, 2871.12, 1704.61, 1611.40, 1582.98, 1509.81, 1458.57, 1425.15, 1378.07, 1362.19, 1297.87, 1239.24, 1220.17, 1176.20, 1116.28, 1023.80, 997.46, 923.19, 823.53, 521.37.

### 1-(Allyloxy)-6-methoxybenzene (1c)

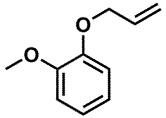

Guaiacol (13.5 mL, 120.7 mmol), K₂CO₃ (37.0 g, 267.7 mmol), allyl bromide (12 mL, 138.7 mmol) in acetone (500 mL) were reacted for a time period of 17 h, resulting in a light-yellow oil (19.9 g, 99% yield) which was isolated without further purification.

¹H NMR (400 MHz, CDCl₃): δ 6.99 -6.84 (m, 4H), 6.10 (ddt, J = 17.2, 10.6, 5.4 Hz, 1H), 5.41 (dq, J = 17.3, 1.6 Hz, 1H), 5.28 (dq, J = 10.5, 1.5 Hz, 1H), 4.62 (dt, J = 5.5, 1.5 Hz, 2H), 3.88 (s, 3H) ppm.

¹³C-APT NMR (101 MHz, CDCl₃): δ 149.65, 148.17, 133.58, 121.37, 120.87, 118.03, 113.74, 111.91, 69.99, 56.01 ppm.

IR-ATR (cm⁻¹): 3065.66, 3002.17, 2936.96, 2835.65, 1648.34, 1591.61, 1501.59, 1454.15, 1439.20, 1423.48, 1327.99, 1248.36, 1221.86, 1178.10, 1122.67, 1052.15, 1022.68, 994.64, 925.02, 832.89, 772.15, 738.72, 577.47.

### General Procedure 2 to obtain the compound of formula (Illa): Claisen Rearrangement

The obtained product of General Procedure 1 was added to a 20 mL ACE pressure tube under nitrogen flow. The tube was sealed and heated to 180 °C for 24 to 60 h, after which it was cooled to room temperature, and either used as-is, or purified further by column chromatography.

### 2-Allyl-6-methoxy-4-propylphenol (2a)

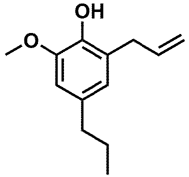

1a (16.4 g, 90.7 mmol) was heated for 60 h, resulting in a dark orange oil (16.3 g, 99% yield) which was isolated without further purification.

¹H NMR (400 MHz, CDCl₃): δ 6.56 (d, J = 3.0 Hz, 2H), 6.01 (ddt, J = 16.8, 10.1, 6.6 Hz, 1H), 5.52 (s, 1H), 5.07 (m, 2H), 3.87 (s, 3H), 3.38 (d, J = 6.3, 1.6 Hz, 1H), 2.49 (t, J = 7.7 Hz, 2H), 1.60 (h, J = 7.5 Hz, 2H), 0.93 (t, J = 7.3 Hz, 3H) ppm.

¹³C-APT NMR (101 MHz, CDCl₃): δ 146.25, 141.37, 137.00, 134.04, 125.43, 122.04, 115.43, 122.04, 115.43, 56.13, 38.00, 34.07, 25.05, 14.00 ppm.

IR-ATR (cm⁻¹): 3543.63, 2957.91, 2930.89, 2870.70, 1638.54, 1604.84, 1497.11, 1463.19, 1434.58, 1366.24, 1287.79, 1233.53, 1211.20, 1148.71, 1069.82, 944.82, 908.26, 837.19.

### 2-Allyl-4-propylphenol (2b)

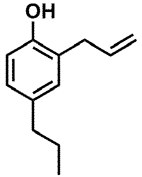

1b (0.92 g, 5.3 mmol) was heated for 24 h resulted in full conversion to a yellow oil (0.84 g, 90% yield), which was isolated without further purification.

¹H NMR (400 MHz, CDCl₃): δ 6.96 - 6.89 (m, 2H), 6.73 (d, J = 8.0 Hz, 1H), 6.02 (ddt, J = 16.8, 10.2, 6.4 Hz, 1H), 5.21 - 5.10 (m, 2H), 4.77 (s, 1H), 3.39 (dt, J = 6.3, 1.7 Hz, 2H), 2.49 (t, J = 7.9 Hz, 2H), 1.59 (t, J = 7.6 Hz, 3H), 0.92 (t, J = 7.3 Hz, 3H) ppm.

¹³C-APT NMR (101 MHz, CDCl₃): δ 152.00, 136.55, 135.13, 130.37, 127.64, 124.83, 116.32, 115.58, 37.15, 35.24, 24.78, 13.79 ppm.

IR-ATR (cm⁻¹): 3448.52, 3078.70, 3010.26, 2960.69, 2929.14, 2871.41, 1638.47, 1611.21, 1503.43, 1434.69, 1377.67,1338.70, 1259.79, 1229.11, 1187.07, 1092.08, 1017.82, 912.51, 868.46, 797.55.

EA: calc; C: 81.77%, H: 9.15%, found; C: 81.61%, 81.31%, H: 9.43, 9.55, average found; C: 81.46%, H: 9.49%, σ; C: 0.212%, H: 0.085%.

### 2-Allyl-6-methoxyphenol (2c)

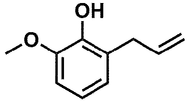

Heating 1c (19.7 g, 119.7 mmol) for 24 h resulted in full conversion to a light brown oil. Purification was performed by column chromatography using pentanes : EtOAc 9:1 over silica. (12.9 g, 66% yield, 82% spectroscopic purity according to ¹H NMR).

¹H NMR (400 MHz, CDCl₃): δ 6.84 - 6.66 (m, 3H), 6.03 (ddt, J = 17.0, 10.1, 6.7 Hz, 1H), 5.13 - 5.04 (m, 2H), 3.89 (s, 3H), 3.43 (dt, J = 6.5, 1.5 Hz, 2H) ppm.

¹³C-APT NMR (101 MHz, CDCl₃): δ 146.52, 143.54, 136.80, 126.01, 122.39, 119.53, 115.54, 108.79, 56.19, 33.95 ppm.

IR-ATR (cm⁻¹): 3517.33, 3076.92, 3057.14, 3005.26, 2962.44, 2907.81, 2841.41, 1732.64, 1638.58, 1617.41, 1592.28, 1476.35, 1440.71, 1356.42, 1265.39, 1215.51, 1068.55, 909.91, 826.59, 737.15.

### General Procedure 3 to obtain the compound of formula (Ila): Esterification

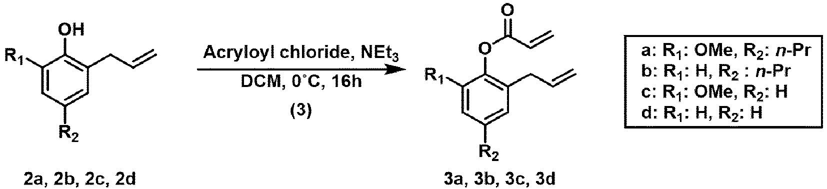

Under the protection of an inert N₂ atmosphere, acryloyl chloride (2 eq., 0.43 M) was added by a syringe to a 3-neck round bottom flask with dry DCM, and was subsequently cooled to 0 °C. A mixture of the product of General Procedure 2 (1 eq., 0.21 mol⁻¹ L) and NEt₃ (3 eq., 0.65 M) in dry DCM was added dropwise to the reaction mixture, evolving white fumes, with subsequent darkening of the reaction mixture to orange, with gradual formation of a yellow/white precipitate. The reaction mixture was left to stir overnight (18 to 20 h) at 0 °C. The mixture was dissolved into additional DCM. It was washed with water and once with brine. The organic layer was dried with MgSO₄, filtered, and concentrated in vacuo, resulting in the desired product.

### 2-Allyl-6-methoxy-4-propylphenyl acrylate (3a)

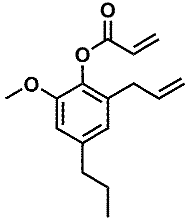

2a (7.0 g, 33.9 mmol), NEt₃ (14 mL, 160.4 mmol) in dry DCM (50 mL) was added to acryloyl chloride (5.5 mL, 68.0 mmol) in dry DCM (100 mL) and reacted for 20 h at 0 °C. Column chromatography with 29:1 pentanes : EtOAc on basic alumina was performed, yielding the resulting product as a colourless oil (5.6 g, 84 % yield).

¹H NMR (400 MHz, CDCl₃): δ 6.68 - 6.63 (m, 2H), 6.60 (dd, J = 17.3, 1.3 Hz, 1H), 6.35 (dd, J = 17.4, 10.4 Hz, 1H), 5.99 (dd, J = 10.4, 1.3 Hz, 1H), 5.87 (ddt, J = 16.8, 10.0, 6.6 Hz, 1H), 5.04 (m, 2H), 3.79 (s, 3H), 3.25 (dt, J = 6.7, 1.7 Hz, 2H), 2.55 (t, J = 7.8 Hz ,2H), 1.64 (h, J = 7.7 Hz, 2H), 0.96 (t, J = 7.4 Hz, 3H) ppm.

¹³C-APT NMR (101 MHz, CDCl₃): δ 164.10, 151.14, 141.29, 136.16, 135.92, 132.89, 132.55, 127.81, 121.74, 116.17, 110.72, 56.07, 38.38, 34.76, 24.69, 14.05 ppm.

IR-ATR (cm⁻¹): 3078.52, 3006.29, 2959.28, 2932.87, 2870.94, 1745.05, 1635.76, 1596.29, 1488.04, 1463.90, 1426.68, 1402.09, 1293.40, 1247.11, 1197.41, 1142.16, 1112.27, 1069.29, 1018.71, 983.44, 911.51, 894.76, 838.97, 799.09.

ESI-MS (CH₃CN): calculated for [M⁺ HNEt₃⁺]: 363.27 m/z, found 362.2 m/z.

### 2-Allyl-4-propylphenyl acrylate (3b)

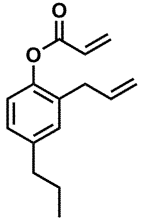

2b (1.7 g, 9.6 mmol), NEt₃ (4 mL, 28.7 mmol), acryloyl chloride (1.75 mL, 21.7 mmol) in dry DCM (45 mL) were reacted for 18 at 0 °C. The organic solution was washed with water (3 x 50 mL) and brine (50 mL), reduced in vacuo, then the product, a yellow oil, was isolated without further purification for the following reaction (2.2 g, 77% yield). For additional, optional purification, a basic alumina column pentanes : EtOAc 9:1 resulted in a colourless oil.

¹H NMR (400 MHz, CDCl₃): δ 7.09 (dq, J = 4.5, 2.2 Hz, 2H), 7.01 (d, J = 8.7 Hz, 1H), 6.62 (dd, J = 17.3, 1.4 Hz, 1H), 6.35 (dd, J = 17.3, 10.5 Hz, 1H), 6.01 (dd, J = 10.4, 1.3 Hz, 1H), 5.92 (ddt, J = 17.5, 9.5, 6.6 Hz 1H), 5.08 (dh, J = 13.6, 2.2, 2H), 3.31 (d, J = 6.6 Hz, 1H), 2.59 (t, J = 7.9, 2H), 1.67 (h, J = 7.6 Hz, 2H), 0.98 (t, J = 7.3 Hz, 3H) ppm.

¹³C-APT NMR (101 MHz, CDCl₃): δ 164.62, 146.79, 140.68, 136.10, 132.40, 131.47, 130.36, 128.01, 127.46, 122.00, 116.19, 37.57, 34.74, 24.62, 13.93 ppm.

IR-ATR (cm⁻¹): 3079.30, 3036.89, 3007.97, 2959.73, 2930.35, 2871.73, 1743.14, 1637.01, 1495.57, 1402.05, 1293.00, 1249.13, 1193.21, 1148.72, 1120.86, 1020.00, 983.34, 907.98, 821.13, 801.24, 733.11.

### 2-Allyl-6-methoxyphenyl acrylate (3c)

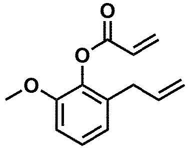

2c (2 g, 12.68 mmol) and NEt₃ (3 mL, 34.4 mmol), acryloyl chloride (2.2 mL, 27 mmol) in dry DCM (10 mL) were reacted for 20 h at 0 °C. The organic solution was washed with water (3 x 250 mL) and brine (250 mL), filtered over a basic alumina plug, dried with MgSO₄ and reduced in vacuo. The crude was then purified by column chromatography over silica (EtOAc:pentanes, 2:8). The product was isolated as an orange oil (2.04 g, 76 % yield).

¹H NMR (400 MHz, CDCl₃): δ 7.18 - 7.13 (m, 1H), 6.90 - 6.82 (m, 2H), 6.62 (dd, J = 17.3, 1.3 Hz, 1H), 6.37 (dd, J = 17.3, 10.4 Hz, 1H) 6.01 (dd, J = 10.5, 1.4 Hz, 1H), 5.88 (ddt, J = 16.9, 10.3, 6.6 Hz, 1H), 5.09 - 5.02 (m, 2H), 3.81 (s, 3H), 3.30 (dt, J = 6.6, 1.6 Hz, 2H) ppm.

¹³C-APT NMR (101 MHz, CDCl₃): δ 163.89, 151.49, 138.09, 135.95, 133.59, 132.53, 127.72, 126.57, 121.90, 116.32, 110.50, 56.12, 34.67 ppm.

IR-ATR (cm⁻¹): 3007.16, 2962.51, 2939.29, 2909.19, 2840.08, 1744.10, 1635.51, 1622.3, 1608.72, 1584.37,1477.64, 1439.35, 1402.31, 1272.82, 1246.70, 1145.28, 1082.29, 1065.84, 1017.67, 984.00, 895.57, 913.43,799.29, 779.96, 751.41.

### 2-Allylphenyl acrylate (3d)

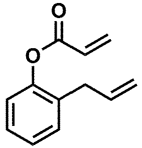

Commercially available 2-allylphenol (10.3 g, 76.5 mmol), NEt₃ (16.5 mL, 118.4 mmol), acryloyl chloride (10 mL, 123.7 mmol) in dry DCM (360 mL) were reacted for 18 h at 0 °C. After the organic layer was washed with water (3 x 350 mL) and brine (350 mL) the resulting product was filtered over a basic alumina plug, followed by drying with MgSO₄, filtering, and concentration in vacuo, resulting in a yellow oil with a pungent odour (12.7 g, 88% yield).

¹H NMR (400 MHz, CDCl₃): δ 7.31 -7.16 (m, 2H), 7.10 -7.07 (dd, J = 8.2, 1.5 Hz, 1H), 6.62 (m, 1H), 6.34 (dd, J = 17.3, 10.5 Hz, 1H), 6.02 (dd, J = 10.5, 1.4 Hz, 1H), 5.90 (ddt, J = 17.7, 9.1, 6.6 Hz, 1H), 5.07 (t, J = 1.5 Hz, 1H), 5.04 (dq, J = 7.2, 1.7 Hz, 1H), 3.31 (dt, J = 6.7, 1.7 Hz, 2H) ppm.

¹³C-APT NMR (101 MHz, CDCl₃): δ 164.53, 148.92, 135.92, 132.68, 132.11, 130.52, 127.98, 127.56, 126.36, 122.44, 116.42, 34.75 ppm.

IR-ATR (cm⁻¹): 3079.11, 3036.68, 3008.09, 2979.95, 2911.48, 1739.25, 1635.04, 1624.00, 1583.63, 1488.21, 1453.78, 1401.95, 1293.42, 1249.32, 1213.20, 1145.56, 118.74, 1019.16, 982.79, 912.91, 800.98, 752.11, 675.87.

### General Procedure 4 to obtain the compound of formula (a): Ring Closing Metathesis

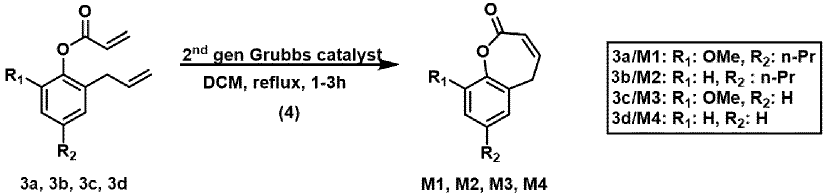

Under an inert N₂ atmosphere, the acrylate ester from General Procedure 3 (1 eq., 4.00 mM) was added to a three-neck round bottom flask with dry DCM which was then stirred and heated to 60 °C. Separately, to Grubbs 2^{nd} generation catalyst (0.5 -1 mol%) in a Schlenk flask, was added minimal dry DCM to dissolve it for immediate cannula transfer to the reaction mixture. After 1 to 3.5 h, the mixture was cooled to room temperature, activated charcoal was added, and the solution was filtered. The filtrate was then concentrated in vacuo.

### 9-Methoxy-7-propylbenzoxepi-2(5H)-one (M1; "MPBOP")

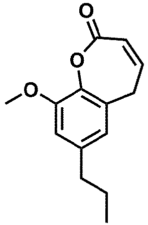

3a (1.66 g, 4.00 mmol) and Grubbs 2^{nd} generation catalyst (26.9 mg, 0.032 mmol) in dry DCM (1 L) were reacted for 1 h. The product was isolated as a yellow oil (1.27 g, 86 % yield).

¹H NMR (400 MHz, CDCl₃): δ 6.89 (dt, J = 11.0, 6.6 Hz, 1H), 6.67 (d, J = 1.9 Hz, 1H), 6.52 (d, J = 1.9 Hz, 1H), 5.92 (dt, J = 11.0, 0.9 Hz, 2H), 3.86 (s, 3H), 3.43 (d, J = 6.6 Hz, 2H), 2.54 (t, J = 7.6, 2H), 1.63 (h, J = 7.5, 2H), 0.94 (t, J = 7.3 Hz, 3H) ppm.

¹H NMR (400 MHz, CD₃CN): δ 6.97 (dt, J = 11.0, 6.7 Hz, 1H), 6.81 (d, J = 2.2 Hz, 1H), 6.62 (d, J = 2.0 Hz, 1H), 5.84 (dt, J = 11.0, 0.9 Hz, 2H), 3.82 (s, 3H), 3.43 (d, J = 6.6 Hz, 2H), 2.54 (t, J = 7.7 Hz 2H), 1.62 (h, J = 7.5 Hz, 2H), 0.92 (t, J = 7.3 Hz, 3H) ppm.

¹³C-APT NMR (101 MHz, CDCl₃): δ 164.10, 151.14, 141.29, 136.16, 135.92, 132.89, 132.55, 127.81, 121.74, 116.17, 110.72, 56.07, 38.38, 34.76, 24.69, 14.05 ppm.

IR-ATR (cm⁻¹): 2959.08, 2931.87, 2869.75, 1724.56, 1597.49, 1488.80, 1463.02, 1379.09, 1349.39, 1288.27, 1241.68, 1206.41, 1184.25, 1140.68, 1091.82, 1078.70, 990.98, 907.01, 817.14, 546.47.

EA: calc; C: 72.39%, H: 6.94%, found; C: 72.63%, 72.76%, H: 7.38%, 7.40%, average found; C: 72.70%, H: 7.39%, σ; C: 0.092%, H: 0.014%.

### 7-Propylbenzoxepi-2(5H)-one (M2; "PBOP")

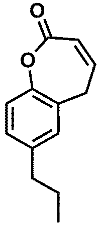

3b (0.76 g, 3.3 mmol), Grubbs 2^{nd} generation catalyst (33 mg, 1.2 mol%, 0.039 mmol) in dry DCM (750 mL) were reacted for 3.5 h. The product was isolated as a light-yellow oil (1.0 g, 76 % yield).

¹H NMR (400 MHz, CDCl₃): δ 7.13 (d, J = 8.2 Hz, 1H), 7.05 (dd, J = 8.2, 2.2 Hz, 1H), 6.94 (d, J = 1.7 Hz, 1H), 6.90 (dt, J = 11.1, 6.6 Hz, 1H), 5.92 (dt, J = 11.1, 0.9 Hz, 1H), 3.44 (d, J = 6.7 Hz, 2H), 2.54 (t, J = 7.8 Hz, 2H), 1.61 (h, J = 14.8, 7.4 Hz, 1H), 0.93 (t, J = 7.3 Hz, 3H) ppm.

¹³C-APT NMR (101 MHz, CDCl₃): 164.18, 149.32, 147.25, 140.28, 131.45, 128.77, 128.23, 121.97, 120.94, 37.28, 31.24, 24.62, 13.87 ppm.

IR-ATR (cm⁻¹): 3044.41,2959.36,2929.53, 2870.10, 1722.11, 1493.62, 1464.22, 1438.00, 1379.90, 124.89, 1201.82, 1188.00, 1162.04, 1141.37, 1104.57, 987.29, 926.51, 893.97, 821.74, 467.56.

EA: calc; C: 77.20%, H: 6.98%, found; 77.50%, 77.24%, H: 6.98%, 7.47%, average found; C: 77.37%, H: 7.23%, σ; C: 0.184%, H: 0.346%.

### 9-methoxybenzoxepi-2(5H)-one (M3; "MBOP")

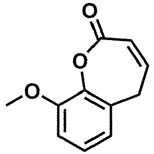

3c (1.6 g, 7.4 mmol), Grubbs 2^{nd} generation catalyst (34 mg, 0.55 mol%, 0.041 mmol) in dry DCM (1.5 L) were reacted for 3 h. Column chromatography with 9:1 pentanes: EtOAc on silica was performed, yielding an impure light-yellow oil. The product was purified by dissolution in hot DCM, followed by a small addition of PE. First a brown solid precipitated which was filtered off and discarded. Subsequently, the product was crystallised from reheating the solution with careful addition of a small quantity (a few mL) of PE and then allowing the solvents to evaporate. The product was isolated as a colourless crystalline solid (1.09 g, 86% yield).

¹H NMR (400 MHz, CDCl₃): δ 7.07 (dd, J = 8.3, 7.6 Hz, 1H), 6.91 (dt, J = 10.9, 6.6 Hz, 1H), 6.86 (dd, J = 8.2, 1.5 Hz, 1H), 6.72 (ddt, J = 7.6, 1.4, 0.7 Hz, 1H), 5.93 (dt, J = 11.0, 0.9 Hz, 1H), 3.88 (s, 3H), 3.47 (dt, J = 6.7, 0.8 Hz, 2H) ppm.

¹³C-APT NMR (101 MHz, CDCl₃): δ 163.50, 151.38, 147.47, 140.38, 133.90, 125.94, 122.00, 120.10, 111.52, 56.24, 31.13 ppm.

IR-ATR (cm⁻¹): 3044.62, 3014.09, 2977.61, 2946.93, 2845.10, 1727.06, 1608.39, 1583.70, 1479.96, 1456.00, 1442.37, 1376.44, 1308.64, 1278.65, 1240.32, 1196.53, 1173.19, 1146.27, 1084.00, 1072.75, 992.23, 977.78, 913.10, 817.37, 778.71, 764.00, 737.11, 651.90, 530.66.

EA: calc; C: 69.46%, H: 5.30%, found; C: 69.62%, 69.54%, H: 5.20%, 5.19%, average found; C: 69.58%, H: 5.20%, σ; C: 0.057%, 0.007%.

### Benzoxepi-2(5H)-one (M4; "BOP")

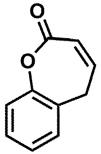

3d (1.6 g, 8.6 mmol), Grubbs 2^{nd} generation catalyst (40 mg, 0.56 mol%, 0.048 mmol) in dry DCM (1.4 L) were reacted for 3 h. Column chromatography with 9:1 pentanes : EtOAc on silica was performed, yielding a white solid. Recrystallisation was achieved by dissolving the sample in a minimal amount of DCM, adding hexane, and allowing the solvents to slowly evaporate, yielding a colourless crystalline solid in quantitative yield (1.51 g measured. This is above the theoretical 100% of 1.41 g but was due to residual DCM when weighing. Subsequent drying removed the solvent allowing for clarified NMR spectra).

¹H NMR (400 MHz, CDCl₃): δ 7.31 - 7.20 (m, 2H), 7.19 - 7.09 (m, 1H), 6.91 (dt, J = 11.1, 6.7 Hz, 1H), 5.95 (dt, J = 11.1, 0.9 Hz, 1H), 3.49 (dd, J = 6.7, 0.9 Hz, 2H) ppm.

¹³C-APT NMR (101 MHz, CDCl₃): δ 163.67, 151.19, 147.08, 131.73, 128.68, 128.30, 125.55, 121.85, 121.15, 31.05 ppm.

IR-ATR (cm⁻¹): 3408.23, 3047.98, 2971.92, 2909.25, 1706.40, 1605.68, 1581.97, 1484.15, 1449.47, 1390.88, 1251.01, 1226.71, 1193.51, 1153.46, 1095.64, 984.21, 918.71, 834.45, 782.18, 754.37, 732.77, 671.34, 615.18, 564.60.

### Hydrogenation of MPBOP (M1)

### 9-Methoxy-7-propyl-4,5-dihydrobenzo[b]oxepi-2(3H)-one (MPBOP-H₂)

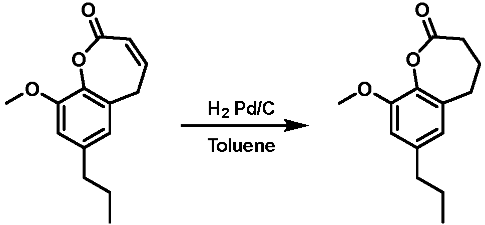

Under the protection of an inert N₂ atmosphere, Pd/C (18.6 mg, 0.175 mmol) was added to toluene (0.5 mL). MPBOP (48.5 mg, 0.209 mmol) in toluene (0.5 mL) was added to the reaction mixture. After removing the nitrogen atmosphere by pulling vacuum on the headspace, the reaction mixture was opened to a hydrogen atmosphere (1 atm) and left to stir overnight. Residual hydrogen from the headspace was removed under vacuum and the vessel was cycled between N₂ and vacuum two more times. The reaction mixture was then filtered over silica and concentrated in vacuo, resulting in a light-yellow oil (42.1 mg, 86%).

Safety note: It is important to re-charge the headspace with nitrogen after completion of the reaction, to prevent potential ignition of the organic solvent upon returning to normal atmospheric composition.

¹H NMR (400 MHz, CDCl₃): δ 6.63 (dd, J = 43.9, 1.9 Hz, 1H), 3.84 (s, 1H), 2.77 (t, J = 7.3 Hz, 1H), 2.54 (t, J = 7.9 1H), 2.47 (t, J = 7.2 Hz, 1H), 2.16 (p, J = 7.2 Hz, 1H), 1.64 (h, J = 7.3 Hz, 1H), 0.95 (t, J = 7.3 Hz, 1H) ppm. ¹³C-APT NMR (101 MHz, CDCl₃): δ 171.81, 149.33, 141.05, 138.65, 131.04, 120.96, 111.72, 56.15, 38.01, 31.23, 28.41, 26.63, 24.65, 13.95 ppm.

IR-ATR (cm⁻¹): 2957.09, 2933.25, 2868.34, 1756.83, 1594.57, 1485.24, 1463.18, 1451.97, 1429.78, 1353.17, 1342.77, 1301.02, 1204.96, 1124.59, 1097.72, 977.40, 838.16, 768.97, 559.68.

General Procedure 5 to obtain the polymer comprising repeating units of formula (Villa) in which - - - is a double bond: Ring Opening Metathesis Polymerisation

The obtained monomers M1 to M4 (MPBOP, PBOP, MBOP, or BOP) were polymerized in a Ring Opening Metathesis Polymerisation (ROMP) to obtain unsaturated polyesters poly-M1 to poly-M7.

A representative synthetic route is provided for a [M]:[cat] of 50:1. In a glovebox, bicyclic monomer (MPBOP, PBOP, MBOP, or BOP), toluene and Grubbs 3^{rd} generation catalyst were added to a Schlenk flask ([MX]:[cat] 50:1, [MX] = 3 M). The Schlenk flask was removed from the glovebox and placed in a preheated oil bath at 60 °C for 1.5 h, resulting in a brown-red reaction mixture. Ethyl vinyl ether in DCM (10% v/v 2 mL) was added to quench the polymerisation. The reaction mixture was dried by rotary evaporation, dissolved into minimal DCM and added dropwise to rapidly stirring cold methanol to precipitate the polymer. The suspension was then transferred to a 50 mL falcon tube and centrifuged at 6000 rpm for 10 min. The green supernatant was then removed, and the white precipitate was resuspended in 5 mL of MeOH, recentrifuged, then isolated and dried in vacuo.

### pMPBOP ("poly-M1")

MPBOP (M1; 149.0 mg, 0.641 mmol), toluene (215 µL) and Grubbs 3^{rd} generation catalyst (8.1 mg, 0.0092 mmol) were stirred for 3 h, resulting in a white powder (42.4 mg).

¹H NMR (400 MHz, CDCl₃): δ 7.21 (dt, J = 15.4, 6.7 Hz, 1H), 6.64 (dd, J = 11.3, 1.9 Hz, 2H), 6.03 (d, J = 15.6 Hz, 1H), 3.75 (s, 2H), 3.43 (d, J = 7.1 Hz, 1H), 2.52 (t, J = 7.8 Hz, 2H), 1.62 (h, J = 6.3 Hz, 2H), 0.93 (t, J = 7.2 Hz, 3H) ppm.

¹³C-APT NMR (101 MHz, CDCl₃): δ 164.21, 151.31, 148.66, 141.45, 136.19, 130.61, 121.76, 121.53, 111.31, 56.05, 38.18, 33.06, 24.65, 14.05 ppm.

IR-ATR (cm⁻¹): 2958.45, 2932.17, 2869.70, 1738.92, 1651.03, 1596.86, 1489.27, 1463.53, 1425.99, 1322.28, 1268.93, 1245.68, 1197.20, 1135.45, 1094.22, 981.63, 838.04.

### pPBOP ("poly-M2")

PBOP (M2; 79.5 mg, 0.393 mmol), toluene (140 µL) and Grubbs 3^{rd} generation catalyst (10.6 mg, 0.012 mmol) were stirred for 24 h, resulting in a white powder.

¹H NMR (400 MHz, CDCl₃): δ 7.21 (dt, J = 15.5, 6.7 Hz, 1H), 7.09 -6.99 (m, 2H), 6.96 (d, J = 8.3 Hz, 1H), 5.97 (d, J = 15.5 Hz, 1H), 3.43 (d, J = 6.8 Hz, 2H), 2.53 (t, J = 7.8 Hz, 2H), 1.59 (h, J = 6.0 Hz, 2H), 0.92 (t, J = 7.2 Hz, 3H) ppm.

¹³C-APT NMR (101 MHz, CDCl₃): δ 164.75, 148.90, 146.97, 140.96, 130.52, 129.17, 128.24, 122.45, 121.71, 37.55, 33.26, 29.84, 13.99 ppm.

IR-ATR (cm⁻¹): 2959.72, 2936.11, 2840.22, 1737.36, 1650.65, 1608.89, 1585.32, 1478.77, 1439.83, 1330.63, 1311.13, 1273.86, 1273.86, 1190.28, 1167.99, 113.95, 1079.57, 977.84, 769.35, 734.13.

### pMBOP ("poly-M3")

MBOP (M3; 70.0 mg, 0.368 mmol), toluene (120 µL) and Grubbs 3^{rd} generation catalyst (6.8 mg, 0.77 µmol) were stirred for 1.5 h, resulting in a white powder (50.4 mg).

¹H NMR (400 MHz, CDCl₃): δ 7.21 (dt, J = 15.6, 6.7 Hz, 1H), 7.12 (t, J = 8.0 Hz, 1H), 6.82 (t, J = 6.5 Hz, 2H), 6.02 (d, J = 15.6 Hz, 1H), 3.74 (s, 3H), 3.45 (d, J = 6.9 Hz, 2H) ppm.

¹³C-APT NMR (101 MHz, CDCl₃): δ 163.92, 151.72, 148.60, 138.33, 131.22, 126.74, 121.94, 121.55, 111.12, 56.10, 33.02 ppm.

IR-ATR (cm⁻¹): 3013.52, 2939.05, 2839.51, 1737.99, 1650.65, 1608.89, 1585.32, 1478.89, 1439.95, 1330.09, 1311.13, 1273.84, 1244.66, 1168.03, 1133.86, 1080.14, 977.92, 948.87, 769.15, 734.08.

### pBOP ("poly-M4")

BOP (M4; 83.2 mg, 0.519 mmol), toluene (170 µL) and Grubbs 3rd generation (10.2 mg, 11.5 µmol) were stirred for 24 h, resulting in a white powder (14.8 mg).

¹H NMR (400 MHz, CDCl₃): δ 7.20 (dq, J = 15.1, 7.7 Hz, 4H), 7.05 (d, J = 8.0 Hz, 1H), 5.96 (d, J = 15.6 Hz, 1H), 3.46 (d, J = 6.6 Hz, 2H) ppm.

¹³C-APT NMR (101 MHz, CDCl₃): δ 164.49, 149.08, 148.89, 130.64, 129.66, 128.37, 126.54, 122.88, 121.73, 33.21 ppm.

IR-ATR (cm⁻¹): 3454.60, 3063.07, 2915.22, 2848.17, 1728.66, 1649.20, 1490.20, 1450.29, 1330.63, 1258.72, 1217.66, 1168.28, 1142.89, 979.70, 795.69, 707.51, 476.97.

The properties of the obtained polymers are shown in Table 1.

**Table 1. Polymer properties of pMPBOP, pPBOP, pMBOP and pBOP.**

| | Mₙ ^{[1]} [kg/mol] | M_{w} ^{[1]} [kg/mol] | Ð ^{[2]} [--] | T_{g} ^{[3]} [°C] | T_{d,10%} ^{[4]} [°C] |
|---|---|---|---|---|---|
| pMPBOP | 13.9 | 17.2 | 1.2 | 76 | 286 |
| pPBOP | 9.2 | 11.6 | 1.3 | 102 | 273 |
| pMBOP | 16.4 | 27.2 | 1.7 | 110 | 293 |
| pBOP | 12.9 | 19.6 | 1.5 | 62 | 267 |

| | | | | | |
|---|---|---|---|---|---|
| [1] Determined by GPC in DMF at 65 °C [2] Polydispersity = M_{w}/Mₙ [3] T_{g} = glass transition temperature; determined by MDSC [4] T_{d,10%} = decomposition temperatures, defined by a 10% weight loss; determined by TGA | | | | | |

As can be seen from the data shown in Table 1, high molecular weight polymers with narrow dispersities were obtained (Ð < 1.7), i.e. relatively monodisperse polymers. All polymers showed similarly high thermal stabilities (see the T_{d,10%} values). The polymers showed T_{g} which are comparable to T_{g}'s of polyamide-6 (PA6) which are typically about 60 °C.

### Hydrogenation of pMPBOP ("poly-M1") to obtain pMPBOP-H₂ ("poly-M1-H2")

Under the protection of an inert N₂ atmosphere, Pd/C (24.4 mg, 0.229 mmol) was added to toluene (1.5 mL). pMPBOP (25.7 mg) in toluene (1.0 mL) was added to the reaction mixture. After removing the nitrogen atmosphere by pulling vacuum on the headspace, the reaction mixture was opened to a hydrogen atmosphere (1 atm) and left to stir overnight. Residual hydrogen from the headspace was removed under vacuum and the vessel was cycled between N₂ and vacuum two more times. The reaction mixture was then filtered over cotton and concentrated in vacuo. The polymer was precipitated by dissolving the crude product in minimal DCM and adding methanol, forming a white suspension, after concentrating in vacuo a white solid was obtained (26.2 mg).

¹H NMR (400 MHz, CDCl₃): δ 6.67 (d, J = 1.8 Hz, 1H), 6.62 (d, J = 2.1 Hz, 1H), 3.75 (s, 3H), 2.65 - 2.54 (m, 2H), 2.52 (t, J = 7.7 Hz, 2H), 1.99 (p, J = 7.1 Hz, 2H), 1.61 (h, J = 7.4 Hz, 1H), 0.93 (t, J = 7.3 Hz, 3H) ppm. ¹³C-APT NMR (100 MHz, CDCl₃): δ 171.71, 150.94, 141.01, 136.30, 134.24, 121.91, 110.47, 55.91, 38.25, 33.45, 29.48, 25.54, 24.69, 14.04 ppm.

IR-ATR (cm⁻¹): 2955.39, 2924.31, 2868.80, 2855.90, 1756.01, 1724.33, 1595.33, 1491.50, 1465.18, 1454.75, 1427.25, 1371.53, 1331.70, 1288.59, 1269.17, 1201.02, 1173.23, 1130.55, 1097.94, 1037.26, 836.98, 803.56, 728.18.

The properties of pMPBOP and its hydrogenation produce pMPBOP-H₂ are shown in Table 2.

**Table 2. Polymer properties of pMPBOP and pMPBOP-H₂**

| | Mₙ ^{[1]} [kg/mol] | M_{w} ^{[1]} [kg/mol] | Ð ^{[2]} [--] | T_{g} ^{[3]} [°C] | T_{d,10%} ^{[4]} [°C] |
|---|---|---|---|---|---|
| pMPBOP | 13.9 | 17.2 | 1.2 | 76 | 286 |
| pMPBOP-H₂ | 14.2 | 17.3 | 1.2 | 27 | 261 |

| | | | | | |
|---|---|---|---|---|---|
| [1] Determined by GPC in DMF at 65 °C [2] Polydispersity = M_{w}/Mₙ [3] T_{g} = glass transition temperature; determined by MDSC [4] T_{d,10%} = decomposition temperatures, defined by a 10% weight loss; determined by TGA | | | | | |

¹H NMR clearly confirmed the loss of the olefinic resonances and appearance of two new aliphatic resonances. Expectedly, hydrogenation of the polymer did not significantly influence the molecular weight, however T_{g} was reduced from 76 °C (pMPBOP) to 27 °C (pMPBOP-H₂).

## Claims

1. A compound of formula (I) 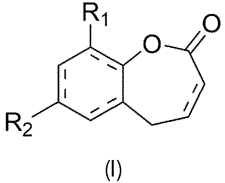 wherein
R₁ and R₂ are independently H, C₁-C₁₈-alkyl or C₁-C₁₈-alkoxy, which alkyl and/or alkoxy is unsubstituted or substituted with at least one substituent R₃,
R₃ is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted,
- - - denotes a single bond or a double bond, and
with the proviso that the compound in which R₁ is H and R₂ is H is excluded.

2. The compound of claim 1, wherein R₁ and R₂ are independently H, C₁-C₁₂-alkyl or C₁-C₁₂-alkoxy, preferably H, C₁-C₃-alkyl or C₁-C₃-alkoxy.

3. The compound of claim 1 or 2, wherein
the alkoxy of R₃ is substituted with at least one substituent selected from C₁-C₁₈-alkoxy and halogen, preferably C₁-C₁₂-alkoxy and halogen, more preferably C₁-C₃-alkoxy and halogen, and
the cycloalkyl, cycloalkoxy, heterocycloalkyl, heterocycloalkoxy, aryl, aryloxy, hetaryl or hetaryloxy of R₅ is substituted with at least one substituent selected from C₁-C₁₈-alkyl, C₁-C₁₈-alkoxy and halogen, preferably C₁-C₁₂-alkyl, C₁-C₁₂-alkoxy and halogen, more preferably C₁-C₃-alkyl, C₁-C₃-alkoxy and halogen.

4. A method for preparing a compound of formula (la) 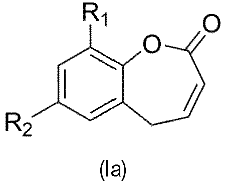 wherein
R₁ and R₂ are independently H, C₁-C₁₈-alkyl or C₁-C₁₈-alkoxy,
R₃ which alkyl and/or alkoxy is unsubstituted or substituted with at least one substituent R₃, and is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted,
with the proviso that the compound in which R₁ is H and R₂ is H is excluded
the method comprising subjecting a compound of formula (IIa) 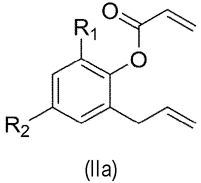
to reaction conditions conducive of a ring-closing metathesis reaction to obtain the compound of formula (la).

5. The method of claim 4, comprising reacting a compound of formula (Illa) 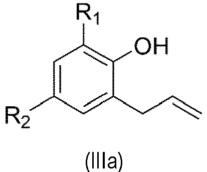
with a compound of formula (lVa) 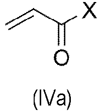
wherein
X is a leaving group, preferably halogen such as Cl or Br, preferably Cl, or acryloyloxy,
to obtain the compound of formula (Ila).

6. The method of claim 5, comprising subjecting a compound of formula (Va) 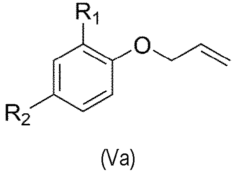 to reaction conditions conducive of a Claisen rearrangement to obtain the compound of formula (Illa).

7. The method of claim 6, comprising reacting a compound of formula (Vla) 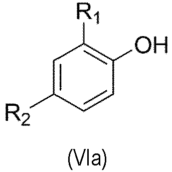
with a compound of formula (VIIa) 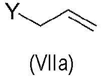
wherein
Y a is leaving group, preferably halogen such as Cl or Br, preferably Br, mesylate or tosylate,
to obtain the compound of formula (Va).

8. The method of claim 7, comprising depolymerizing lignin to obtain the compound of formula (Vla).

9. The method of any one of claims 4 to 8, comprising hydrogenating the compound of formula (la) to obtain the compound of formula (lb)

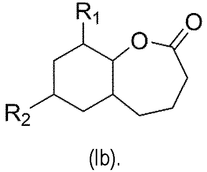

10. A polymer comprising repeating units of formula (Villa) 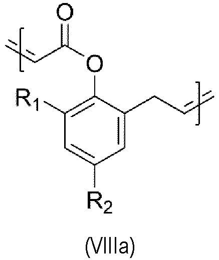 wherein
R₁ and R₂ are independently H, C₁-C₁₈-alkyl or C₁-C₁₈-alkoxy, which alkyl and/or alkoxy is unsubstituted or substituted with at least one substituent R₃,
R₃ is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy,
- - - which alkoxy, cycloalkyl, cycloalkoxy, heterocycloalkyl, heterocycloalkoxy, aryl, aryloxy, hetaryl or hetaryloxymay be unsubstituted or substituted, and denotes a single bond or a double bond.

11. A method for preparing the polymer of claim 10 in which - - - is a double bond, comprising subjecting the compound of formula (I) of claim 1 to reaction conditions conducive of a ring-opening metathesis polymerization reaction to obtain the polymer comprising repeating units of formula (Villa) in which - - - is a double bond.

12. The method of claim 11, wherein reaction conditions conducive of a ring-opening metathesis polymerization reaction comprise adding a metathesis catalyst, which metathesis catalyst is preferably selected from (C-1), (C-2) and (C-3):

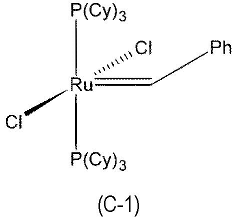

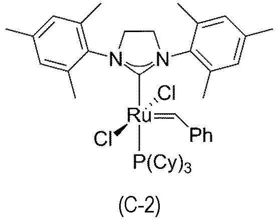

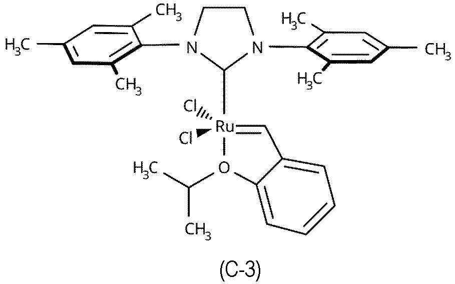

13. The method of claim 11, comprising hydrogenating the compound of formula (VIIIa) in which - - - is a double bond to obtain a compound of formula (Villa) in which - - - is a single bond.

14. A polymer comprising repeating units of formula (IX) 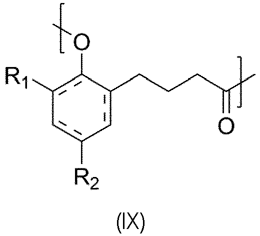 wherein
R₁ and R₂ are independently H, C₁-C₁₈-alkyl or C₁-C₁₈-alkoxy, which alkyl and/or alkoxy is unsubstituted or substituted with at least one substituent R₃,
R₃ is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted, and
- - - denotes a single bond or a double bond.

15. A method for preparing the polymer of claim 14, comprising subjecting a compound of formula (I') 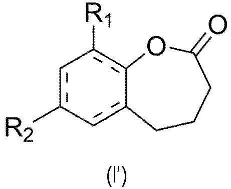 wherein
R₁ and R₂ are independently H, C₁-C₁₈-alkyl or C₁-C₁₈-alkoxy, which alkyl and/or alkoxy is unsubstituted or substituted with at least one substituent R₃,
R₃ is independently halogen, hydroxyl, C₁-C₁₈-alkoxy, C₃-C₈-cycloalkyl, C₃-C₈-cycloalkoxy, 3 to 8-membered heterocycloalkyl, 3 to 8-membered heterocycloalkoxy, C₅-C₁₀-aryl, C₅-C₁₀-aryloxy, 3 to 8-membered hetaryl or 3 to 8-membered hetaryloxy, which alkoxy, cycloalkyl, cycloalkoxy, heterocycloalkyl, heterocycloalkoxy, aryl, aryloxy, hetaryl or hetaryloxy may be unsubstituted or substituted, and
- - - denotes a single bond or a double bond,
to reaction conditions conducive of a ring-opening polymerization reaction to obtain the polymer comprising repeating units of formula (IX).
